# EUROPEAN PATENT APPLICATION

(11) **EP 2 583 693 A1**
(43) Date of publication of application: **24.04.2013**
(21) Application number: 11798001.1
(22) Date of filing: 13.06.2011
(51) Int. Cl.: A61K 48/00, A01K 67/027, A61K 35/76, A61P 3/10, A61P 43/00, C12N 5/10, C12N 15/00, C12N 15/09, C12Q 1/02, C12Q 1/48

(54) **PHARMACEUTICAL COMPOSITION UTILIZING PANCREATIC CELL PROLIFERATION FACTOR**

(30) Priority: 21.06.2010 JP 2010140444
(71) Applicant: Tokyo Institute of Technology, Tokyo 152-8550 (JP)
(72) Inventor: IMAI, Takeshi, Yokohama-shi Kanagawa 226-8503 (JP); HANDA, Hiroshi, Yokohama-shi Kanagawa 226-8503 (JP)
(74) Representative: Woods, Geoffrey Corlett
(86) International application number: PCT/JP2011/063459
(87) International publication number: WO 2011/162115

(57) **Abstract**

Disclosed are a pharmaceutical composition, a screening method, and the like which use UDP-glucose glycoprotein glycosyl transferase 1 (UGGT1) or a gene encoding the same. UGGT1 has an extremely high proliferative activity compared to known pancreatic β-cell proliferation factors; thus, UGGT1 can act as a useful therapeutic agent for diabetes without any modification and is also useful as a target substance for the development of a new therapeutic agent for diabetes.

SEQUENCE LISTING

<110> Tokyo Institute of Technology

<120> A PHARMACEUTICAL COMPOSITION UTILIZING PANCREATIC BETA CELL GROWTH
FACTOR

<130> FP-156PCT

<150> JP 2010/140444
<151> 2010-06-21

<160> 2

<170> PatentIn Ver. 2.1

<210> 1
<211> 23
<212> DNA
<213> Artificial Sequence

<220>
<223> Description of Artificial Sequence:primer

<400> 1
gctctgactg accgcgttac tcc                                         23


<210> 2
<211> 21
<212> DNA
<213> Artificial Sequence

<220>
<223> Description of Artificial Sequence:primer

<400> 2
gggagtgtcc caggaatcag g                                           21

## Description

### TECHNICAL FIELD

The present invention relates to a pharmaceutical composition, a screening method, and the like which use UDP-glucose glycoprotein glycosyl transferase 1 (hereinafter referred to as "UGGT1") or a gene encoding the same. UGGT1 has an extremely high proliferative activity compared to known pancreatic β-cell proliferation factors; thus, UGGT1 can act as a useful therapeutic agent for diabetes without any modification and is also useful as a target substance for the development of a new therapeutic agent for diabetes.

### BACKGROUND ART

Diabetes is a today's most prevalent disease in Japan (the number of sufferers are expected to be on the order of 14.7 million inclusive of potential sufferers), and mostly occurs due to insulin hyposecretion for Japanese sufferers. Langerhans islet β-cells in the pancreas, the sole source secreting insulin, particularly in diabetic patients are few in number, and the medicine for regenerating the β-cells is very important.

A plurality of regenerating factors increasing pancreatic β-cells are present which have been known to date. Examples thereof include Pdx1 (Non-Patent Documents 1 and 2), Mafa (Non-Patent Document 3), Ngn3 (Non-Patent Document 3), GLP-1 (Non-Patent Document 4), hepatic erk (Non-Patent Document 5), and osteocalcin (Non-Patent Document 6).

### NON-PATENT DOCUMENT

Non-Patent Document 1
   Rui Takahashi et al., Journal of Molecular Endocrinology 38: 127-136, 2007.
Non-Patent Document 2
   Junta Imai et al., Biochemical and Biophysical Research Communications 326: 4022-409, 2005.
Non-Patent Document 3
   Qiao Zhou et al., Nature 455: 627-633, 2008.
Non-Patent Document 4
   Doris A Stoffers et al., Diabetes 49: 741-748, 2000.
Non-Patent Document 5
   Junta Imai et al., Science 322: 1250-1254, 2008.
Non-Patent Document 6
   Na Kyung Lee et al., Cell 130: 456-469, 2007.

### DISCLOSURE OF THE INVENTION

### PROBLEM TO BE SOLVED BY THE INVENTION

The above-described pancreatic β-cell proliferation factors only increase pancreatic β-cells on the order of 2 to 3 times as compared to a wild-type one. There is a need for a factor higher in the proliferative activity for the treatment of diabetes. Made under such a technical background, the present invention has an object of providing a pancreatic β-cell proliferation factor having a high proliferative activity.

### MEANS FOR SOLVING THE PROBLEM

As the result of intensive studies for solving the above-described problems, the present inventors have found that a protein called UGGT1 has a proliferative activity on pancreatic β-cells and that the proliferative activity thereof is significantly higher than known pancreatic β-cell proliferation factors.

Although the sequences of UGGT1 and the gene thereof are known, it has not been known that the protein has a proliferative activity on pancreatic β-cells. Mice with knockout of UGGT1 gene have been prepared (Reference 1); however, the analysis of the pancreas has not been carried out because they die during the fetal period.

The present invention has been accomplished based on the above findings.

Specifically, the present invention provides the following (1) to (8).
(1) A pharmaceutical composition comprising a vector for expressing UGGT1 gene.
(2) The pharmaceutical composition according to (1), wherein the composition is used for the prevention or treatment of diabetes.
(3) A method comprising administering the pharmaceutical composition according to (1) or (2) to a non-human animal and proliferating pancreatic β-cells in the non-human animal.
(4) A transgenic non-human animal, wherein UGGT1 gene is introduced thereinto and the gene is expressed.
(5) A cell transformed with a vector in which a reporter gene is linked downstream of a promoter of UGGT1 gene.
(6) The cell according to (5), wherein the cell is a pancreatic β-cell.
(7) A screening method for a preventive agent or a therapeutic agent for diabetes, comprising the steps of culturing the cell according to (5) or (6) in the presence of each test substance; and selecting a test substance having increased the expression level of the reporter gene.
(8) A screening method for a preventive agent or a therapeutic agent for diabetes, comprising the steps of mixing each test substance with UGGT1 and selecting test substances interacting with UGGT1; and administering each of the selected test substances to a non-human animal and selecting a test substance proliferating pancreatic β-cells in the non-human animal.

### ADVANTAGE OF THE INVENTION

The UGGT1 contained in the pharmaceutical composition of the present invention has a high proliferative activity on pancreatic β-cells. Thus, the pharmaceutical composition of the present invention is useful as a preventive agent or a therapeutic agent for diabetes and the like. UGGT1 is also useful as target substance for the development of a therapeutic agent for diabetes.

### BRIEF DESCRIPTION OF DRAWINGS

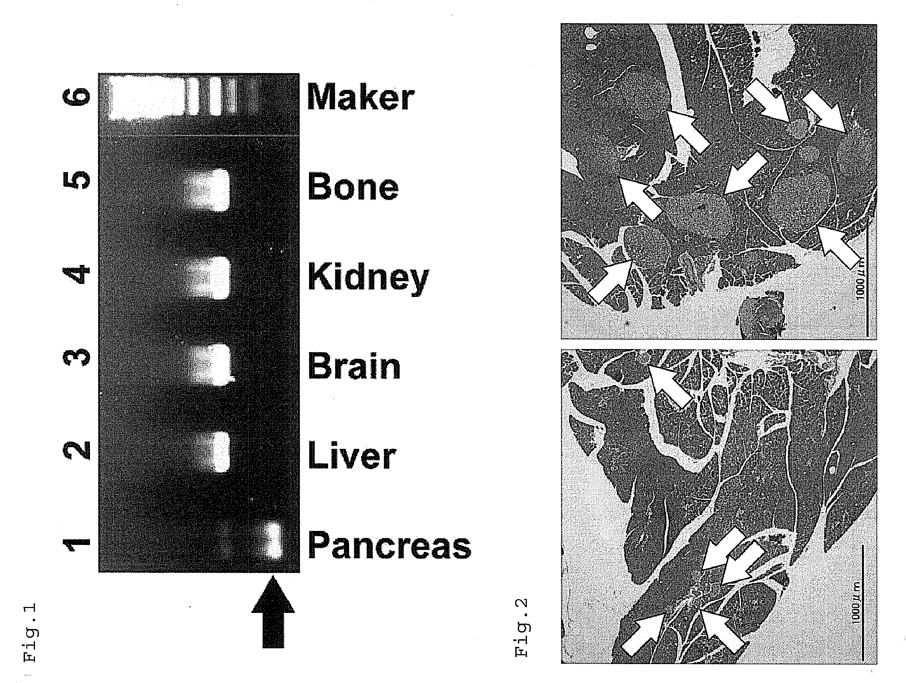

[Figure 1] Figure 1 is a photograph showing the results of analyzing the amount of mRNA for UGGT gene in each organ of a mouse in which the UGGT1 gene is forcedly expressed in pancreatic β-cells (hereinafter simply referred to as "UGGT1 mouse") by a RT-PCR method. RT-PCR was performed on RNA extracted from the pancreas (lane 1), the liver (lane 2), the brain (lane 3), the kidney (lane 4), and the bone (lane 5). The mRNA for UGGT1 gene is detected at the position indicated by the arrow. As shown in the figure, the mRNA for UGGT1 gene was detected only when RT-PCR was carried out on the RNA extracted from the pancreas;
[Figure 2] Figure 2 is a pair of microscope photographs of the pancreas in a wild-type mouse (left) and a UGGT1 mouse (right). The arrow indicates islets of Langerhans. The scale bar on the lower left indicates 1,000 µm in each photograph;

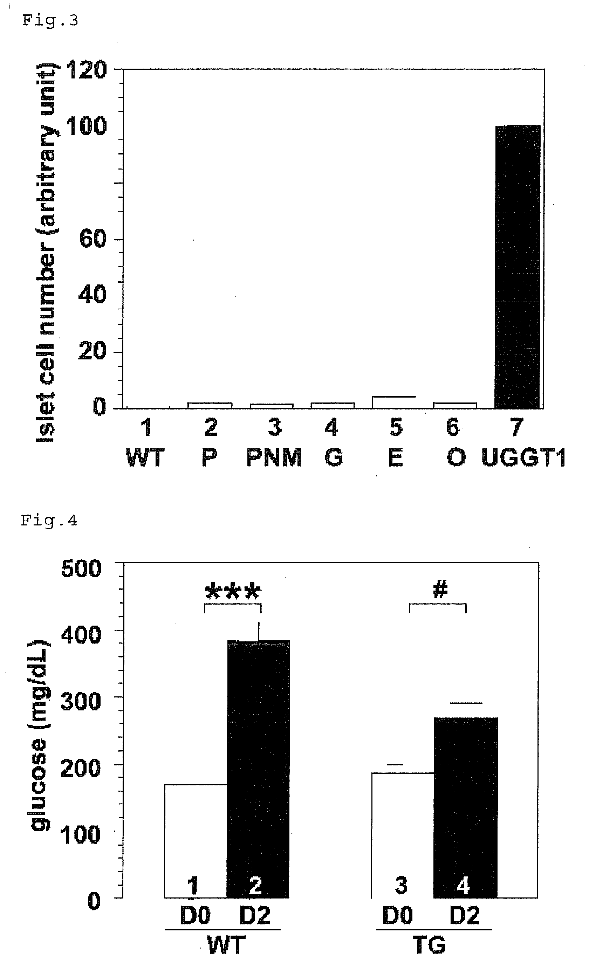

[Figure 3] Figure 3 is a graph showing the size of islets of Langerhans of a variety of mice. In the figure, 1 WT indicates a wild-type mouse; 2 P, a mouse into which Pdx1 gene was introduced (References 2 and 3); 3 PNM, a mouse into which Pdx1 gene, Ngn3 gene, and Mafa gene were introduced (Reference 4); 4 G, a mouse into which GLP1 gene was introduced (Reference 5); 5 E, a mouse in which erk gene was introduced into the liver (Reference 6); 6 O, a mouse into which osteocalcin gene was introduced (Reference 7); and 7 UGGT1, a UGGT1 mouse;
[Figure 4] Figure 4 is a graph showing changes in the blood glucose levels of wild-type and UGGT1 mice to which streptozotocin was administered. Blood was collected immediately before (D0) and 48 hours after (D2) administering streptozotocin to the wild-type (WT) and UGGT1 (TG) mice and the blood glucose level thereof was measured. The streptozotocin administration significantly increased the blood glucose level in the wild-type mice (p<0.0001), while no significant increase was observed in the UGGT1 mice (#>0.05); and

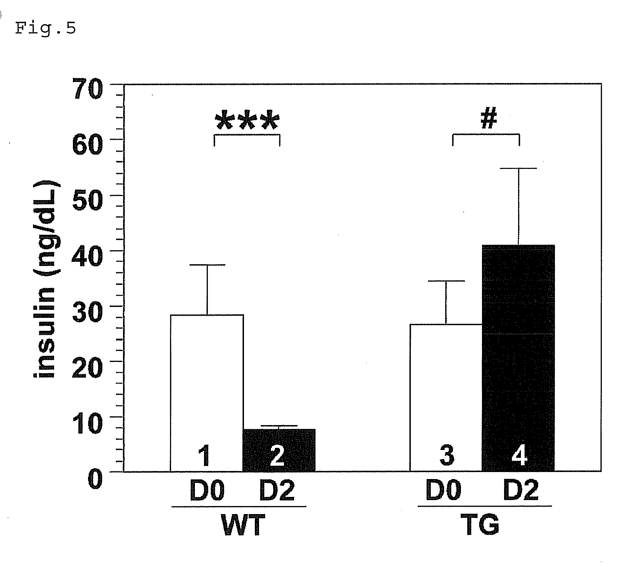

[Figure 5] Figure 5 is a graph showing changes in the insulin concentration in wild-type and UGGT1 mice to which streptozotocin was administered. Blood was collected immediately before (D0) and 48 hours after (D2) administering streptozotocin to the wild-type (WT) and UGGT1 (TG) mice and the insulin concentration thereof was measured. The streptozotocin administration significantly decreased the insulin concentration in the wild-type mice (***; p<0.0001), while no significant decrease was observed in the UGGT1 mice (#; p>0.05).

### BEST MODE FOR CARRYING OUT THE INVENTION

The present invention will be described below in detail.

The pharmaceutical composition of the present invention comprises a vector for expressing UGGT1 gene.

UGGT1 is a known protein, and the nucleotide sequence of the gene encoding the same (UGGT1 gene) is also publicly available on data bases such as NCBI and GenBank. For example, the nucleotide sequences of mouse-derived UGGT1 gene and human-derived UGGT1 gene are registered under accession numbers NM_198899 and NM_020120, respectively. The UGGT1 protein and UGGT1 gene used may be naturally-occurring; however, they may be modified UGGT1 consisting of an amino acid sequence in which one or a plurality of amino acids are deleted , substituted, or added in the amino acid sequence of the naturally-occurring UGGT1 and having a β-cell-proliferating activity like the naturally-occurring UGGT1, and a gene encoding the same.

The UGGT1 gene is used by inserting it into a vector together with an appropriate promoter and the like. As the promoter, the promoter of UGGT1 gene per se may be used, or another promoter may be used. The promoter other than the promoter of UGGT1 gene is preferably one capable of directing the expression of the UGGT1 gene in the pancreas; examples thereof can include insulin II promoter and insulin I promoter.

The vector may be one used for gene therapy, for example, a virus vector. Examples of the virus vector can include adenovirus vector, retrovirus vector, AAV vector, and SV40 vector.

The method for administering the pharmaceutical composition of the present invention is not particularly limited, and may be according to a method used for a typical gene therapy method. For example, the composition may be administered by intradermal, intraperitoneal, intravenous, intraarterial, or intraspinal injection or drip infusion.

The dosage of the pharmaceutical composition of the present invention is not particularly limited, and may be properly selected depending on the dosage form, the route of administration, the properties of a subject for administration, and the like. The typical dosage is suitably in the range of 10 mg to 1,000 mg in the weight of UGGT1 gene per day per adult.

When administered by injection or drip infusion, the pharmaceutical composition of the present invention may contain ingredients typically contained in an injection solution or a drip-feed solution. Examples of such ingredients can include fluid carriers (e.g., potassium phosphate buffer, physiological saline, Ringer injection, distilled water, polyethylene glycol, vegetable oil, ethanol, glycerin, dimethyl sulfoxide, and propylene glycol), antimicrobial agents, local anesthetics (e.g., procaine hydrochloride and dibucaine hydrochloride), buffers (e.g., tris-hydrochloric acid buffer and HEPES buffer), and osmo-regulators (e.g., glucose, sorbitol, and sodium chloride).

The pharmaceutical composition of the present invention can be used for the prevention or treatment of diabetes. The pharmaceutical composition of the present invention is effective on both type-1 diabetes and type-2 diabetes; however, it is expected to be particularly effective on type-1 diabetes since it causes the regeneration of β-cells.

The pharmaceutical composition of the present invention is intended to be used in humans; however, it may be administered to animals other than humans to proliferate pancreatic β-cells in these animals. Subject animals can include, for example, mice, rats, dogs, and monkeys.

The UGGT1 gene can be used for the preparation of a transgenic animal in addition for the pharmaceutical composition. For example, a transgenic animal expressing UGGT1 gene can be prepared by injecting a vector for expressing the UGGT1 gene into a fertilized ovum of an animal other than humans and transplanting the fertilized ovum into a provisional parent. Animals targeted for preparation can include mice, rats, dogs, and monkeys.

Because UGGT1 proliferates pancreatic β-cells, a substance increasing the amount of UGGT1 in a living body (substance A) and a substance enhancing the pancreatic β-cell-proliferating activity of UGGT1 (substance B) can provide candidates for therapeutic agents for diabetes. Thus, a new preventive or therapeutic agent for diabetes can be found by screening for the substance A or the substance B.

Methods for screening for the substance A can include, for example, a method comprising the steps of culturing cells transformed with a vector in which a reporter gene is linked downstream of the promoter of UGGT1 gene, in the presence of each of test substances; and selecting a substance having increased the expression level of the reporter gene.

The substance increasing the expression level of the reporter gene in cells is expected to enhance the activity of the promoter of UGGT1 gene in a living body and also to increase the expression level of the UGGT1 gene in the living body. Thus, the substance obtained by the screening method can provide a preventive or therapeutic agent for diabetes.

The reporter gene used may be one commonly used for screening methods or the like, and may be, for example, GFP gene, luciferase gene, alkaline phosphatase gene, or β-gal gene.

The sequence of the promoter of UGGT1 gene is known and publicly available on data bases. For example, the promoter of mouse-derived UGGT1 gene is present on mouse chromosome 1, and the nucleotide sequence thereof is registered under accession number NT_039170, NM_001030649, or the like; human-derived UGGT1 gene is present on human chromosome 2, and the nucleotide sequence thereof is registered under accession number NT_022135, NW_921507, NW_001838849, or the like; and rat-derived UGGT1 gene is present on rat chromosome 9, and the nucleotide sequence thereof is registered under accession number NW_047814, NW_001084882, or the like.

The preparation of the vector in which a reporter gene is linked downstream of the promoter of UGGT1 gene and the transformation of cells with the vector can be carried out according to an ordinary method. On this occasion, the promoter of UGGT1 gene and the cells used are preferably derived from the same species of organism.

The cells used are preferably pancreatic β-cells. The pancreatic β-cells used may be, for example, NIT-1 cells, MIN6 cells, or RIN5 cells.

Whether the test substance has increased the expression level of the reporter gene can be determined by comparison with the expression level of the reporter gene in the cells cultured in the absence of the test substance.

Methods for screening for the substance B can include, for example, a method comprising the steps of mixing each of test substances with UGGT1 and selecting test substances interacting with UGGT1; and administering each of the above-selected test substances to a non-human animal and selecting a test substance proliferating pancreatic β-cells in the non-human animal.

It is highly probable that substances interacting with UGGT1 are substances influencing the β-cell-proliferating activity of UGGT1; thus, the selection of such substances can narrow the test substances. However, the substances selected here also include a substance interacting with UGGT1 but not influencing the β-cell-proliferating activity thereof and a substance, on the contrary, inhibiting the β-cell-proliferating activity. Accordingly, each of the selected substances can be administered to a non-human animal, followed by examining the proliferation state of pancreatic β-cells in the non-human animal to eliminate the above-described substances to select only a substance enhancing the β-cell-proliferating activity.

Whether interaction with UGGT1 is present or not can be determined according to a method commonly applied to a screening method or the like. Example of the method can include two-hybrid, one-hybrid, FRET, HTRF, and LANCE.

The non-human animal to which a test substance is administered may be an animal similar to the above-described transgenic animal.

### EXAMPLES

The present invention will be described below in further detail with reference to Examples.

### Example 1: Preparation of UGGT1 Mouse

An expression vector was constructed in which UGGT1 gene was incorporated into rat insulin II promoter gene (position 243 to 1,125 of the sequence described in RATINSII registered in GenBank), and injected into a fertilized ovum of a C3H mouse or a C57Black6 mouse, followed by placing the fertilized ovum in a provisional mouse. After the birth of an F0 mouse, the genotype thereof was confirmed using PCR primers 199M (5'-GCTCTGACTGACCGCGTTACTCC-3': SEQ ID NO: 1) and 201M (5'-GGGAGTGTCCCAGGAATCAGG-3': SEQ ID NO: 2), followed by crossing 6 times with C57Black6 mice. The resultant F6 mouse (6 month-old) was subjected to the analysis of the expression of UGGT1 gene by RT-PCR. As a result, the introduced UGGT1 gene was specifically expressed at the RNA level in the pancreas (Figure 1).

When the pancreas of the F6 mouse (6-month old) was subjected to pathological analysis, extremely more (about 100-fold) β-cells were observed to appear in the mouse than those in the wild-type mouse (Figure 2). The appearance of such many β-cells was observed not only in the 6-month old mouse but also 1-, 3-, and 12-month old mice, and also observed not only in the F6 mouse but also in F3, F4, and F5 mice.

The numbers of pancreatic islet cells in the UGGT1 mouse and mice in which known pancreatic β-cell-proliferation factors were expressed are shown in Figure 3. The numbers of the cells were calculated based on the data present in texts, figures, and the like in papers (References 2 to 7) describing the known factors, and expressed as relative values to the number of the cells in wild-type mice in each paper (expressed as 1). As shown in the figure, the numbers of pancreatic islet cells in the mice in which the known factors were expressed were at most on the order of several times those in the wild-type mice, while that in the UGGT1 mouse was on the order of 100 times.

### Example 2: Analysis of Therapeutic Effect of UGGT1 in Diabetic Model Animal

As shown in Figures 2 and 3, the number of pancreatic islet cells was markedly increased in the UGGT1 mouse. Accordingly, the therapeutic effect of UGGT1 was attempted to be analyzed by administering streptozotocin to UGGT1 mice to induce diabetes. Streptozotocin is a reagent inducing diabetes by specifically killing pancreatic β-cells to decrease the concentration of insulin. Blood was collected before administration (D0), blood was further collected 48 hours after administration (D2), and the blood glucose level and the insulin concentration therein were examined.

The blood glucose levels before administration were 200 mg/dL or less in both the wild-type and UGGT1 mice, showing no significant difference. After administration, however, the blood glucose level in the wild-type mice was on the order of 400 mg/dL and significantly higher than that before administration (p<0.0001) and diabetes was induced. On the other hand, the blood glucose level in the UGGT1 mice was on the order of 250 mg/dL and not significantly different from that before administration and diabetes was not induced (Figure 4).

The insulin concentration was significantly decreased after administration in the wild-type mice as expected but not decreased in the UGGT1 mice (Figure 5). These results revealed that the UGGT1 mice were resistant to the killing of β-cells by streptozotocin and demonstrated the anti-diabetic activity of UGGT1 in the mouse body.

### References:

References 1: Maurizio Molinari, Camela Galli, Omar Vanoni, Stacey M Arnold, Randal J Kaufman. Persistent glycoprotein misfolding activities the glucosidase II/UGT1-driveb Calnexin cycle to delay aggregation and loss of folding competence. Molecular Cell vol20, p503-512, 2005
References 2: Rui Takahashi, Hisanitsu Ishuhara, KazumaTakahashi, Akira Tamura, Suguru Yamaguchi, Takahiro Yamada, Hideki Katagiri, Yoshitomo Oka. Efficient and controlled gene expression in mouse pancreatic isltes by arterial delivery of tetracycline-inducible adenoviral vectors. Journal of Molecular Endocrinology 38:127-136, 2007.
References 3: Junta Imai, Hideki Katagiri, Tatsuya Yamada, Yasushi Ishigaki, Takehide Ogihara, Kenji Uno, Yutaka Hasegawa, Junhong Gao, Hisamitsu Ishihara, Hironobu Sasano, Hiroyuki Mizuguchi, Tomoichiro Asano, Yoshitomo Oka. Constitutively active PDX1 induced efficient insulin production in adult murine liver. Biochemical and Biophysical Research Communications 326:4022-409, 2005.
References 4: Qiao Zhou, Juliana Brown, Andrew Kanarek, Jayaraj Rajagopal, Douglas A Melton. In vivo reprogramming of adult pancreatic exocrine cells to b-cells. Nature 455:627-633, 2008.
References 5: Doris A Stoffers, Timothty J Kieffer, Mehboob A Hussain, Daniel J Drucker, Susan Bonner-Weir, Joel F Habener, Josephine M Egan. Insulinotropic Glucagon-like peptide 1 stimulate expression of homeodomain protein IDX-1 and increase islet size in mouse pancreas. Diabetes 49:741-748, 2000.
References 6: Junta Imai, Hideki Katagiri, Tetsuya Yamada, Yasushi Ishigaki, Toshinobu Suzuki, Hirohito Kudo, Kenji Uno, Yutaka Hasegawa, Junhong Gao, Keizo Kaneko, Hisamitsu Ishihara, Akira Niijima, Masamitsu Nakazato, Tomoichiro Asano, Yasuhiro Minokoshi, Yoshitomo Oka. Reluration of pancreatic b cell mass by neuronal signals from the liver. Science 322:1250-1254, 2008.
References 7: Na Kyung Lee, Hideaki Sowa, Eiichi Hinoi, Mathieu Ferron, Jong Deok Ahn, Cyrille Confavreux, Romain Dacquin, Patrick J Mee, Marc D McKee, Dae Young Jung, Zhiyou Zhang, Jason K Kim, Franck Mauvais-Jarvis, Patricia Ducy, Gerard Karsenty. Endocrine regulation of energy metabolism by the skeleton. Cell 130:456-469, 2007.

### INDUSTRIAL APPLICABILITY

The present invention is useful as a preventive agent or a therapeutic agent for diabetes. It is also useful for the development of a new therapeutic agent for diabetes.

The present specification encompasses the contents of the specification and/or drawings of Japanese Patent Application No. 2010-140444 on which the priority of the present application is based. All publications, patents, and patent applications cited in the present specification are intended to be incorporated herein by reference in their entirety.

## Claims

1. A pharmaceutical composition comprising a vector for expressing UGGT1 gene.

2. The pharmaceutical composition according to claim 1, wherein the composition is used for the prevention or treatment of diabetes.

3. A method comprising administering the pharmaceutical composition according to claim 1 or 2 to a non-human animal and proliferating pancreatic β-cells in the non-human animal.

4. A transgenic non-human animal, wherein UGGT1 gene is introduced thereinto and the gene is expressed.

5. A cell transformed with a vector in which a reporter gene is linked downstream of a promoter of UGGT1 gene.

6. The cell according to claim 5, wherein the cell is a pancreatic β-cell.

7. A screening method for a preventive agent or a therapeutic agent for diabetes, comprising the steps of culturing the cell according to claim 5 or 6 in the presence of each test substance; and selecting a test substance having increased the expression level of the reporter gene.

8. A screening method for a preventive agent or a therapeutic agent for diabetes, comprising the steps of mixing each test substance with UGGT1 and selecting test substances interacting with UGGT1; and administering each of the selected test substances to a non-human animal and selecting a test substance proliferating pancreatic β-cells in the non-human animal.
